# EUROPÄISCHE PATENTSCHRIFT

(11) **EP 1 133 497 B1**
(45) Veröffentlichungstag und Bekanntmachung des Hinweises auf die Patenterteilung: **23.02.2005**
(21) Anmeldenummer: 99959278.5
(22) Anmeldetag: 15.11.1999
(51) Int. Cl.: C07D 471/04, A61K 31/4709, A61P 31/04

(54) **KRISTALLMODIFIKATION B VON 8-CYAN- 1-CYCLOPROPYL -7-(1S,6S-2, 8-DIAZABICYCLO - 4.3.0]NONAN -8-YL)-6- FLUOR-1, 4-DIHYDRO- 4-OXO-3- CHINOLINCARBONSAURE**
CRYSTAL MODIFICATION B OF 8-CYANO-1- CYCLOPROPYL -7-(1S,6S-2, 8-DIAZABICYCLO 4.3.0]NONAN -8-YL)-6- FLUORO-1,4- DIHYDRO-4- OXO-3-QUINOLINE CARBOXYLIC ACID
MODIFICATION CRISTALLINE B D'ACIDE 8-CYAN-1-CYCLOPROPYL -7-(1S,6S-2, 8-DIAZABICYCLO 4.3.0]NONAN -8-YL)-6- FLUOR -1,4-DIHYDRO -4-OXO-3- CHINOLINCARBOXYLIQUE

(30) Priorität: 25.11.1998 DE 19854355
(43) Veröffentlichungstag der Anmeldung: 19.09.2001
(73) Patentinhaber: Bayer HealthCare AG, 51368 Leverkusen (DE)
(72) Erfinder: HIMMLER, Thomas, D-51519 Odenthal (DE); HALLENBACH, Werner, D-40789 Monheim (DE); RAST, Hubert, D-51381 Leverkusen (DE)
(86) Internationale Anmeldenummer: PCT/EP1999/008776
(87) Internationale Veröffentlichungsnummer: WO 2000/031076

(56) Entgegenhaltungen:
- WO-A-97/31001

## Beschreibung

Die vorliegende Erfindung betrifft eine definierte Kristallmodifikation von 8-Cyan-1-cyclopropyl-7-(1 S,6S-2,8-diazabicyclo[4.3.0]nonan-8-yl)-6-fluor-1,4-dihydro-4-oxo-3-chinolincarbonsäure, Verfahren zu ihrer Herstellung und ihre Verwendung in pharmazeutischen Zubereitungen.

8-Cyan-1-cyclopropyl-7-(1S,6S-2,8-diazabicyclo[4.3.0]nonan-8-yl)-6-fluor-1,4-dihydro-4-oxo-3-chinolincarbonsäure der Formel (I) soll im folgenden als CCDC bezeichnet werden.

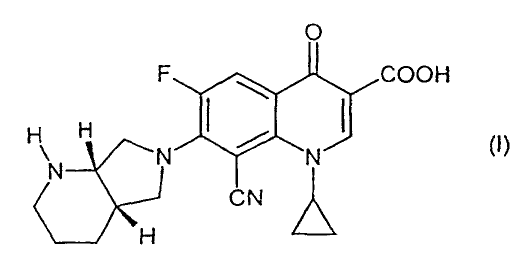

CCDC ist bekannt aus DE-A 19 633 805 oder PCT Anm.-Nr. 97 903 260.4 Sie wird danach hergestellt durch Umsetzung von 7-Chlor-8-cyan-1-cyclopropyl-6-fluor-1,4-dihydro-4-oxo-3-chinolincarbonsäure mit (1S,6S)-2,8-Diazabicyclo[4.3.0]nonan in einem Gemisch aus Dimethylformamid und Acetonitril in Gegenwart einer Hilfsbase. Nach Versetzen mit Wasser wird CCDC mit Dichlormethan aus Wasser extrahiert und durch Entfernen des Extraktionsmittels isoliert. Man erhält dabei ein Pulver, das keine eindeutige Kristallmodifikation aufweist. Das Pulver ist vielmehr zu großen Teilen amorph und kann Gemische verschiedener Kristallmodifikationen enthalten. Sollte durch Zufall eine einheitliche Kristallmodifikation entstehen, ist unklar, wie sie extrahiert und definiert erhalten werden kann. Für die Herstellung von Arzneimitteln ist jedoch Voraussetzung, daß für einen Wirkstoff, der in verschiedenen Kristallmodifikationen vorliegen kann, eindeutig angegeben wird, in welcher Kristallmodifikation er zur Herstellung des Mittels eingesetzt wird.

Das z.T. amorphe Pulver, das nach dem oben skizzierten Herstellverfahren erhalten wird, ist zudem hygroskopisch. Amorphe Feststoffe, und erst recht hygroskopische Feststoffe, sind in der galenischen Verarbeitung jedoch schlecht handzuhaben, da sie beispielsweise geringe Schüttdichten und mangelhafte Fließeigenschaften aufweisen. Außerdem sind zur Handhabung hygroskopischer Feststoffe spezielle Arbeitstechniken und Einrichtungen erforderlich, um reproduzierbare Ergebnisse, z.B. bezüglich des Wirkstoffgehaltes oder der Stabilität in den produzierten Festformulierungen zu erhalten.

Der Erfindung liegt daher die Aufgabe zugrunde, eine kristalline Form definierter Modifikation von CCDC herzustellen, die aufgrund ihrer physikalischen Eigenschaften, insbesondere ihrer Kristalleigenschaften und ihres Verhaltens gegenüber Wasser, in galenischen Formulierungen gut zu handhaben ist.

Diese Aufgabe wird erfindungsgemäß durch eine neue, kristalline Form von CCDC gelöst, die nachstehend als Modifikation B bezeichnet wird.

Gegenstand der Erfindung ist daher die kristalline Modifikation B von CCDC, die dadurch gekennzeichnet ist, daß sie ein Röntgen-Pulverdiffraktogramm mit den in der folgenden Tabelle 1 angegebene Reflexlagen ( 2 Theta) hoher und mittlerer Intensität (> 30% relative Intensität) aufweist.

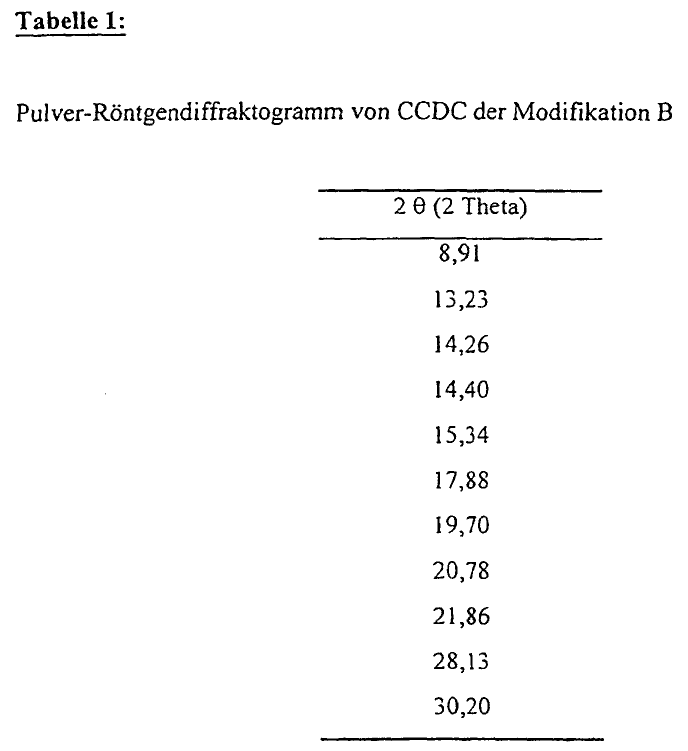

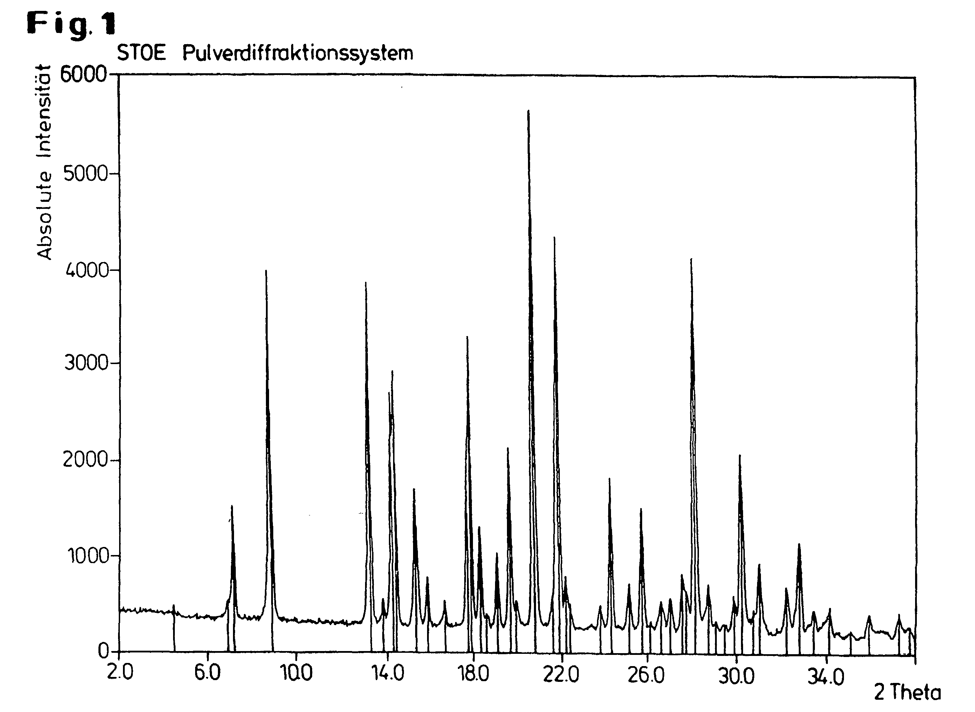

Das Pulver-Röntgendiffraktogramm der Modifikation B ist auch in der Abbildung 1 wiedergegeben.

Die erfindungsgemäße Modifikation B von CCDC unterscheidet sich über außerdem in einer Reihe weiterer Eigenschaften von anderen Formen der CCDC. Auch diese Eigenschaften können einzeln oder gemeinsam mit den übrigen Parametern zur Charakterisierung der erfindungsgemäßen Modifikation B von CCDC dienen.

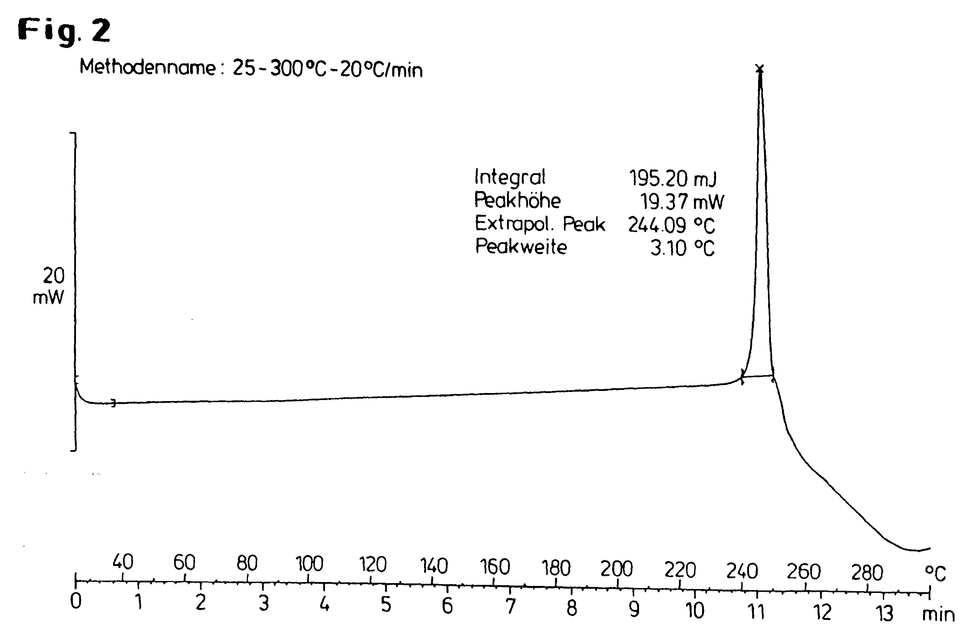

CCDC der Modifikation B ist dadurch gekennzeichnet, daß es einen mit Hilfe der Differentialthermoanalyse (DTA) bestimmten Schmelzpunkt von 243°C bis 245°C hat. Ein charakteristisches Differentialthermadiagramm ist in der Abbildung 2 wiedergegeben.

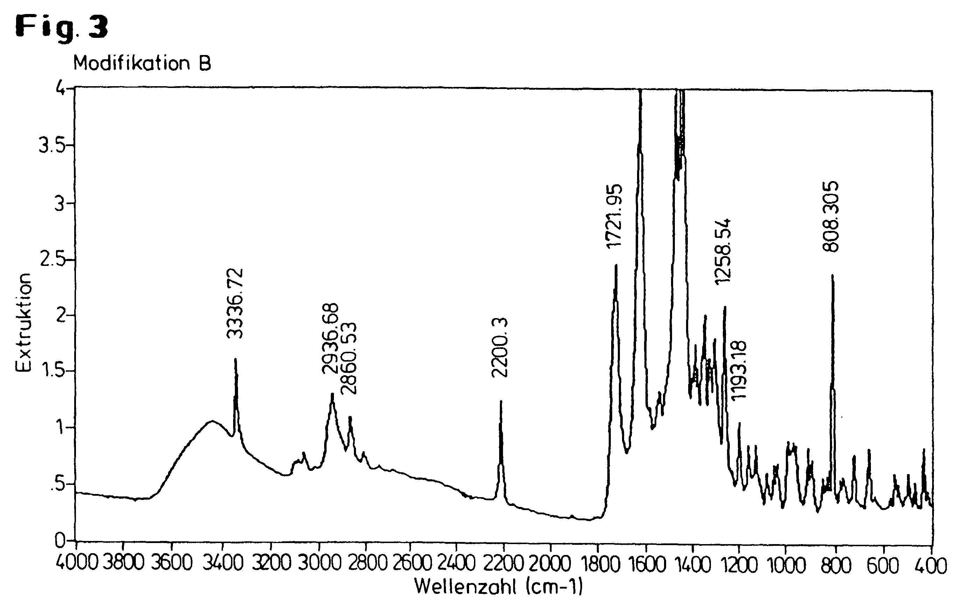

CCDC der Modifikation B ist dadurch gekennzeichnet, daß es ein in KBr gentessenes Infrarotspektrum wie in Abbildung 3 gezeigt besitzt.

CCDC der Modifikation B ist dadurch gekennzeichnet, daß es nach einem der im folgenden angegebenen Herstellverfahren erhältlich ist. Die Kristallmodifikation B von CCDC wird dadurch erhalten, daß 7-Halogen-8-cyan-1-cyclopropyl-6-fluor-1,4-dihydro-4-oxo-3-chinoloncarbonsäure der Formel (II) 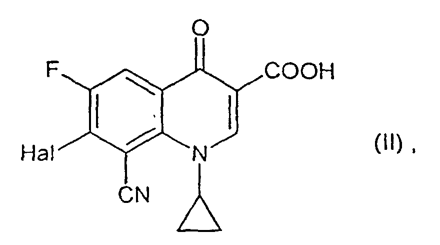 in welcher
- Hal: für Fluor oder bevorzugt für Chlor steht,
und (1S,6S)-2,8-Diazabicyclo[4.3.0]nonan der Formel (III) 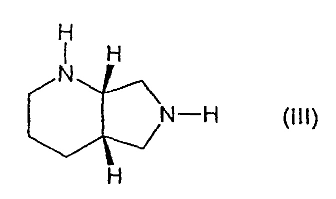 gegebenenfalls in Gegenwart einer Base
in Ethanol in Mischung mit einem polar aprotischen Verdünnungsmittel wie N-Methyl-pyrrolidon, Dimethylformamid und Sulfolan umgesetzt werden,
oder
daß CCDC unbekannter Modifikation in einem Verdünnungsmittel wie Ethanol, Propanol oder Isopropanol oder in einem Gemisch dieser Alkohole mit einem polar aprotischen Verdünnungsmittel wie N-Methyl-pyrrolidon, Dimethylformamid oder Sulfolan gegebenenfalls in Gegenwart einer Base erhitzt wird und anschließend das Gemisch abgekühlt und CCDC der Kristallmodifikation B isoliert wird.

CCDC der Kristallmodifikation B ist überraschend stabil und wandelt sich auch bei längerer Lagerung nicht in eine andere Kristallmodifikation oder die amorphe Form um. Darüber hinaus neigt die Modifikation B im Vergleich mit amorphem CCDC weit weniger zur Aufnahme von Wasser aus der Luft. Es ist aus diesen Gründen hervorragend zur Herstellung von Tabletten oder anderen Festformulierungen geeignet. Durch seine Stabilität verleiht es diesen Formulierungen die gewünschte lang andauernde Lagerstabilität. Mit der Kristallmodifikation B können damit definiert und gezielt stabile feste Zubereitungen von CCDC hergestellt werden.

CCDC der Kristallmodifikation B ist hervorragend gegen pathogene Bakterien auf dem Gebiet der Human- oder Tiermedizin wirksam. Sein breites Einsatzgebiet entspricht dem von CCDC.

Als Basen zur Herstellung von CCDC der Modifikation B werden bevorzugt die tertiären Amine Trimethylamin, Triethylamin, Ethyldiisopropylamin (Hünig-Base), N-Methyl-piperidin, N-Ethyl-piperidin, N-Propyl-piperidin und N-Butyl-piperidin eingesetzt. Ganz besonders bevorzugt sind Triethylamin und Ethyl-diisopropylamin. Auf 1 mol der Verbindung (II) werden normalerweise 1 bis 2 mol Base, bevorzugt 1,1 bis 1,5 mol eingesetzt.

Verwendet man ein Gemisch aus Ethanol und N-Methyl-pyrrolidon, Dimethylformamid und Sulfolan so liegen Ethanol und polar aprotisches Lösungsmittel in Verhältnissen von 0,5 zu 1 bis 4 zu 1 vor; bevorzugt sind Verhältnisse von 1 zu 1 bis 3 zu 1.

Die Umsetzung erfolgt bei Normaldruck oder bei erhöhtem Druck zwischen 1 bar und 100 bar, vorzugsweise zwischen 1 bar und 20 bar.

Die Umsetzung erfolgt bei Temperaturen zwischen 0°C und 200°C, vorzugsweise zwischen 20°C und 150°C.

Auf 1 Mol der Verbindung (II) werden normalerweise 1 bis 2 Mol, vorzugsweise 1 bis 1,5 Mol der Verbindung (III) eingesetzt.

CCDC der Kristallmodifikation B fällt aus der Reaktionsmischung aus und kann abgesaugt werden. Der abgesaugte Feststoff kann noch durch Waschen mit Ethanol gereinigt werden.

Die Ausgangsprodukte der Formeln (II) und (III) zur Herstellung von CCDC sind bekannt (vgl. DE-A 19 633 805).

Wird CCDC unbekannter Modifikation in einem Verdünnungsmittel wie Ethanol, Propanol oder Isopropanol oder in einem Gemisch dieser Alkohole mit einem polar aprotischen Verdünnungsmittel wie N-Methyl-pyrrolidon, Dimethylformamid oder Sulfolan einige Stunden erhitzt, so wird anschließend bei Raumtemperatur abgesaugt, mit Ethanol gewaschen und anschließend getrocknet. Bevorzugt wird auch bei dieser Arbeitsweise als Base Triethylamin oder Ethyl-diisopropylamin zugesetzt (pro 1 Mol Wirkstoff ca. 0,01 bis 0,1 Mol Base).

Das Röntgen-Pulverdiffraktogramm zur Charakterisierung der Kristallmodifikation B von CCDC wurde mit einem Transmissions-Diffraktometer STADI-P mit ortsempfindlichen Detektor (PSD2) der Firma Stoe erhalten.

Der Schmelzpunkt der Differentialthermoanalyse wurde mit dem Gerät DSC 820 der Firma Mettler-Toledo erhalten. Dabei wurde die Probe von CCDC der Kristallmodifikation B in einem Aluminiumtiegel mit 10 K/min an der Luft aufgeheizt. Das IR-Spektrum wurde mit dem Gerät FTS 60A der Firma Biorad in KBr erhalten.

Die folgenden Beispiele illustrieren die Erfindung ohne sie einzuschränken. Die in folgenden Beispielen eingesetzten Lösungsmittel-/Basensysteme sind besonders bevorzugt.

### Vergleichsbeispiel

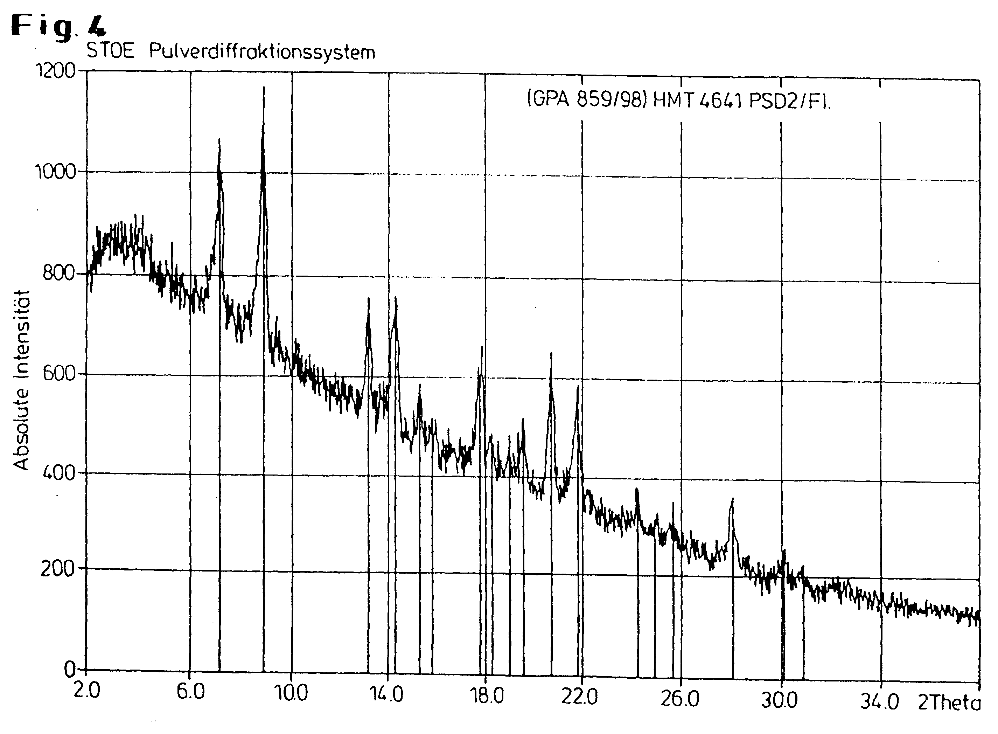

Eine Mischung aus 3,07 g 7-Chlor-8-cyan-1-cyclopropyl-6-fluor-1,4-dihydro-4-oxo-3-chinolincarbonsäure, 1,39 g (1S,6S)-2,8-Diazabicyclo[4.3.0]nonan, 2,24 g 1,4-Diazabicyclo[2.2.2]octan (DABCO), 29,5 ml Dimethylformamid und 29,5 ml Acetonitril wird 16 Stunden bei Raumtemperatur gerührt. Das Reaktionsgemisch wird bei 60°C Badtemperatur am Rotationsverdampfer eingeengt und der Rückstand in 10 ml Wasser aufgenommen. Die resultierende Lösung wird mit verdünnter Salzsäure auf pH 7 gestellt und der Feststoff abfiltriert. Das Filtrat wird dreimal mit je 20 ml Dichlormethan ausgeschüttelt. Man trocknet die organische Phase über Natriumsulfat, filtriert und engt das Filtrat am Rotationsverdampfer bei 60°C Badtemperatur ein. Man erhält 2,4 g hellbraunen Feststoff, der das in der Abbildung 4 gezeigte Röntgen-Pulverdiffraktogramm aufweist und demnach zum großen Teil amorph ist.

Der gemäß dieser Vorschrift erhaltene Feststoff nimmt bei einer relativen Luftfeuchtigkeit von 95 % (eingestellt durch eine gesättigte Lösung von Na₂HPO₄ x 12 H₂O mit Bodensatz in Wasser) innerhalb eines Tages ca. 17 Gewichtsprozent Wasser auf.

### Beispiel 1

1012 g 7-Chlor-8-cyan-1-cyclopropyl-6-fluor-1,4-dihydro-4-oxo-3-chinolincarbonsäure werden in einer Mischung aus 3300 ml Ethanol, 1980 ml N-Methyl-pyrrolidon und 534 g Diisopropylethylamin (Hünig-Base) vorgelegt. Man erhitzt zum Rückfluß und tropft dann 459 g (1S,6S)-2,8-Diazabicyclo[4.3.0]nonan zu. Nach Beendigung des Zutropfens rührt man noch 3 Stunden unter Rückfluß, läßt dann auf Raumtemperatur abkühlen, saugt den Feststoff ab und wäscht ihn mit insgesamt 1800 ml Ethanol.

Der erhaltene Feststoff wird in einer Mischung aus 4650 ml Ethanol und 41 g Hünig-Base suspendiert und das Reaktionsgemisch 3 Stunden zum Rückfluß erhitzt. Man läßt das Reaktionsgemisch wieder auf Raumtemperatur abkühlen, saugt den Feststoff ab, wäscht mit insgesamt 1000 ml EtOH nach und trocknet bei 60 bis 70°C im Vakuumtrockenschrank bis zur Gewichtskonstanz. Man erhält 1130 g beigen Feststoff, der das in der Abbildung 1 gezeigte Röntgen-Pulverdiffraktogramm, das in der Abbildung 2 gezeigte Differentialthermodiagramm und das in der Abbildung 3 gezeigte IR-Spektrum aufweist.

Der gemäß dieser Vorschrift erhaltene Feststoff nimmt bei einer relativen Luftfeuchtigkeit von 95 % (eingestellt durch eine gesättigte Lösung von Na₂HPO₄ x 12 H₂O mit Bodensatz in Wasser) innerhalb eines Tages ca. 1 Gewichtsprozent Wasser auf.

### Beispiel 2

Eine Mischung aus 4,6 g 7-Chlor-8-cyan-1-cyclopropyl-6-fluor-1,4-dihydro-4-oxo-3-chinolincarbonsäure, 15 ml Ethanol, 9 ml N-Methyl-pyrrolidin und 1,9 g Triethylamin wird zum Rückfluß erhitzt. Man tropft 2,08 g (1S,6S)-2,8-Diazabicyclo[4.3.0]nonan zu und rührt dann 3 Stunden bei Rückfluß. Der Feststoff wird bei Raumtemperatur abgesaugt, mit insgesamt 10 ml Ethanol gewaschen und bis zur Gewichtskonstanz getrocknet. Man erhält 5,23 g beigen Feststoff, dessen Differentialthermodiagramm dem der Modifikation B entspricht.

### Beispiel 3

Eine Mischung aus 4,6 g 7-Chior-8-cyan-1-cyclopropyl-6-fluor-1,4-dihydro-4-oxo-3-chinolincarbonsäure, 15 ml Ethanol, 9 ml N-Methyl-pyrrolidin und 2,12 g N-Ethyl-piperidin wird zum Rückfluß erhitzt. Man tropft 2,08 g (1S,6S)-2,8-Diazabicyclo-[4.3.0]nonan zu und rührt dann 3 Stunden bei Rückfluß. Der Feststoff wird bei Raumtemperatur abgesaugt, mit insgesamt 10 ml Ethanol gewaschen und bis zur Gewichtskonstanz getrocknet. Man erhält 5,1 g beigen Feststoff, dessen Differentialthermodiagramm dem der Modifikation B entspricht.

### Beispiel 4

Eine Mischung aus 9,2 g 7-Chlor-8-cyan-1-cyclopropyl-6-fluor-1,4-dihydro-4-oxo-3-chinolincarbonsäure, 30 ml Ethanol, 18 ml Dimethylformamid und 4,85 g Hünig-Base wird zum Rückfluß erhitzt. Man tropft 4,17 g (1S,6S)-2,8-Diazabicyclo[4.3.0]nonan zu und rührt dann 3 Stunden bei Rückfluß. Der Feststoff wird bei Raumtemperatur abgesaugt, mit insgesamt 20 ml Ethanol gewaschen und bis zur Gewichtskonstanz getrocknet. Man erhält 11 g beigen Feststoff, dessen Differentialthermodiagramm dem der Modifikation B entspricht.

### Beispiel 5

Eine Mischung aus 9,2 g 7-Chlor-8-cyan-1-cyclopropyl-6-fluor-1,4-dihydro-4-oxo-3-chinolincarbonsäure, 30 ml Ethanol, 18 ml Sulfolan und 4,85g Hünig-Base wird zum Rückfluß erhitzt. Man tropft 4,17 g (1S,6S)-2,8-Diazabicyclo[4.3.0]nonan zu und rührt dann 3 Stunden bei Rückfluß. Der Feststoff wird bei Raumtemperatur abgesaugt, mit insgesamt 20 ml Ethanol gewaschen und bis zur Gewichtskonstanz getrocknet. Man erhält 10,8 g beigen Feststoff, dessen Differentialthermodiagramm dem der Modifikation B entspricht.

### Beispiel 6

0,5 g des Feststoffes aus dem Vergleichsbeispiel werden in 3 ml Ethanol suspendiert. Das Reaktionsgemisch wird 3 Stunden zum Rückfluß erhitzt, der Feststoff bei Raumtemperatur abgesaugt und getrocknet. Das Röntgen-Pulverdiffraktogramm entspricht dem der Modifikation B.

## Patentansprüche

1. 8-Cyan-1-cyclopropyl-7-(1S,6S-2,8-diazabicyclo[4.3.0]nonan-8-yl)-6-fluor-1,4-dihydro-4-oxo-3-chinolincarbonsäure (CCDC) der Kristallmodifikation B, **dadurch gekennzeichnet, daß** sie ein Röntgen-Pulverdiffraktogramm mit folgenden Reflexlagen (2 Theta) hoher und mittlerer Intensität aufweist

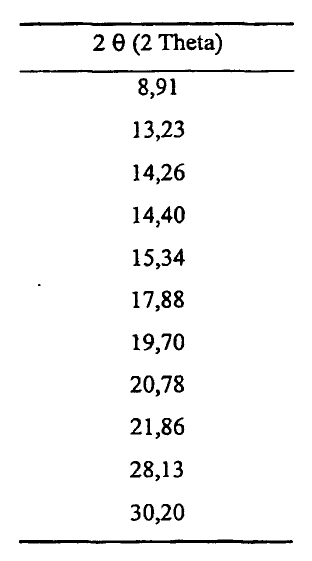

2. 8-Cyan-1-cyclopropyl-7-(1S,6S-2,8-diazabicyclo[4.3.0]nonan-8-yl)-6-fluor-1,4-dihydro-4-oxo-3-chinolincarbonsäure (CCDC) der Kristallmodifikation B, **dadurch gekennzeichnet, daß** sie ein Röntgen-Pulverdiffraktogramm mit folgenden Reflexlagen (2 Theta) hoher und mittlerer Intensität aufweist 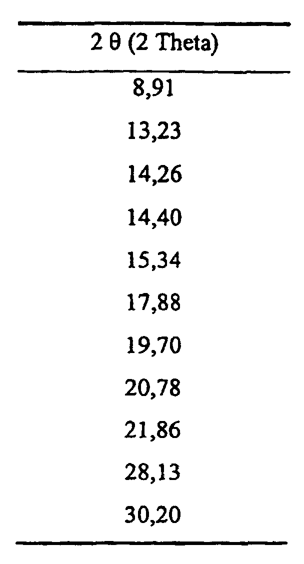 und einen durch DTA ermittelten Schmelzpunkt von 243°C bis 245°C besitzt.

3. 8-Cyan-1-cyclopropyl-7-(1S,6S-2,8-diazabicyclo[4.3.0]nonan-8-yl)-6-fluor-1,4-dihydro-4-oxo-3-chinolincarbonsäure (CCDC) der Kristallmodifikation B, **dadurch** erhältlich daß 7-Halogen-8-cyan-1-cyclopropyl-6-fluor-1,4-dihydro-4-oxo-3-chinoloncarbonsäure der Formel (II) 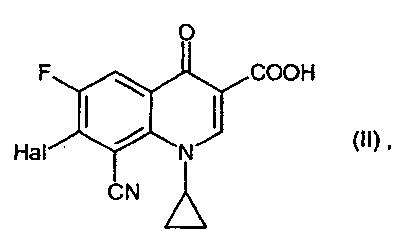 in welcher
Hal für Fluor oder bevorzugt für Chlor steht,
und (1S,6S)-2,8-Diazabicyclo[4.3.0]nonan der Formel (III) 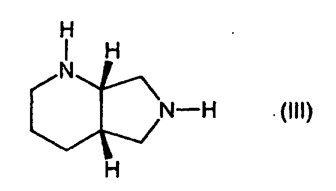 gegebenenfalls in Gegenwart einer Base in
Ethanol in Mischung mit einem polar aprotischen Verdünnungsmittel umgesetzt werden;
oder
daß CCDC unbekannter Modifikation in einem Verdünnungsmittel wie Ethanol, Propanol oder Isopropanol oder in einem Gemisch dieser Alkohole mit einem polar aprotischen Verdünnungsmittel gegebenenfalls in Gegenwart einer Base erhitzt wird und anschließend das Gemisch abgekühlt und CCDC der Kristallmodifikation B isoliert wird.

4. Verfahren zur Herstellung von CCDC der Modifikation B gemäß einem der Ansprüche 1 bis 3, **dadurch gekennzeichnet, daß** 7-Halogen-8-cyan-I-cyclopropyl-6-fluor-1,4-dihydro-4-oxo-3-chinoloncarbonsäure der Formel (II) 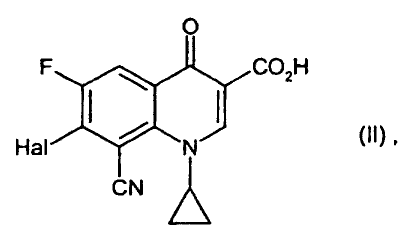 in welcher
Hal für Fluor oder bevorzugt für Chlor steht,
und (1S,6S)-2,8-Diazabicyclo[4.3.0]nonan der Formel (III) 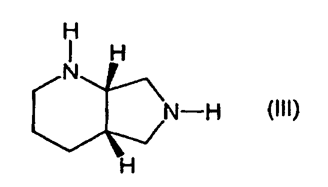 gegebenenfalls in Gegenwart einer Base in
Ethanol in Mischung mit einem polar aprotischen Verdünnungsmittel umgesetzt werden
oder
daß CCDC unbekannter Modifikation in einem Verdünnungsmittel wie Ethanol, Propanol oder Isopropanol oder in einem Gemisch dieser Alkohole mit einem polar aprotischen Verdünnungsmittel gegebenenfalls in Gegenwart einer Base erhitzt wird und anschließend das Gemisch abgekühlt und CCDC der Kristallmodifikation B isoliert wird.

5. Verfahren zur Herstellung von CCDC der Modifikation B gemäß Anspruch 4, **dadurch gekennzeichnet, daß** als polar aprotisches Lösungsmittel N-Methylpyrrolidon, Dimethylformamid oder Sulfolan verwendet wird.

6. Verfahren zur Herstellung von CCDC der Modifikation B gemäß Anspruch 4 oder 5, **dadurch gekennzeichnet, daß** bei Verwendung von Ethanol als Lösungsmittel N-Methyl-pyrrolidon, Dimethylfonnamid oder Sulfolan als weitere Lösungsmittel eingesetzt werden.

7. Verfahren zur Herstellung von CCDC der Modifikation B gemäß einem der Ansprüche 4 bis 6, **dadurch gekennzeichnet, daß** bei Verwendung von Ethanol als Lösungsmittel N-Methyl-pyrrolidon als weiteres Lösungsmittel eingesetzt wird.

8. Verfahren zur Herstellung von CCDC der Modifikation B gemäß einem der Ansprüche 4 bis 7, **dadurch gekennzeichnet, daß** als Base Trimethylamin, Triethylamin oder Ethyl-diisopropylamin eingesetzt werden.

9. Arzneimittel, **dadurch gekennzeichnet, daß** es neben üblichen Hilfs- und Trägerstoffen CCDC der Modifikation B nach einem der Ansprüche 1 bis 3 enthält.

10. Verwendung von CCDC der Modifikation B nach einem der Ansprüche 1 bis 3 zur Herstellung von Arzneimitteln.

11. Verwendung von CCDC der Modifikation B gemäß Anspruch 10 zur Herstellung von antibakteriellen Arzneimitteln.

## Claims

1. 8-Cyano-1-cyclopropyl-7-(1S,6S-2,8-diazabicyclo[4.3.0]nonan-8-yl)-6-fluoro-1,4-dihydro-4-oxo-3-quinolinecarboxylic acid (CCDC) of the crystal modification B, **characterized in that** it has an X-ray powder diffractogram with the following reflection signals (2 theta) of high and medium intensity

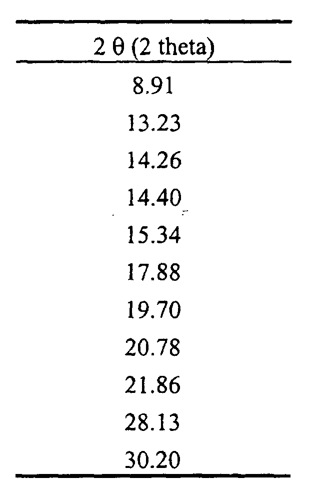

2. 8-Cyano-1-cyclopropyl-7-(1S,6S-2,8-diazabicyclo[4.3.0]nonan-8-yl)-6-fluoro-1,4-dihydro-4-oxo-3-quinolinecarboxylic acid (CCDC) of the crystal modification B, **characterized in that** it has an X-ray powder diffractogram with the following reflection signals (2 theta) of high and medium intensity 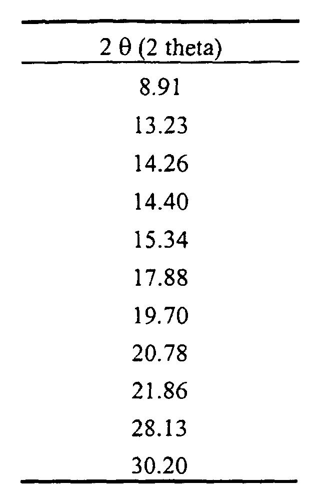 and a melting point, determined by DTA, of from 243°C to 245°C.

3. 8-Cyano-1-cyclopropyl-7-(1S,6S-2,8-diazabicyclo[4.3.0]nonan-8-yl)-6-fluoro-1,4-dihydro-4-oxo-3-quinolinecarboxylic acid (CCDC) of the crystal modification B, obtainable by reacting 7-halogeno-8-cyano-1-cyclopropyl-6-fluoro-1,4-dihydro-4-oxo-3-quinolinecarboxylic acid of the formula (II) 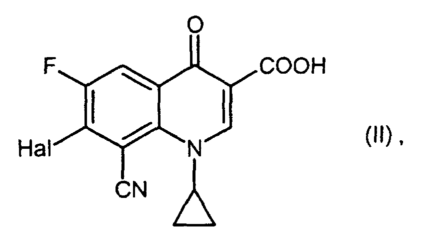 in which
Hal represents fluorine or, preferably, represents chlorine
and (1S,6S)-2,8-diazabicyclo[4.3.0]nonane of the formula (III) 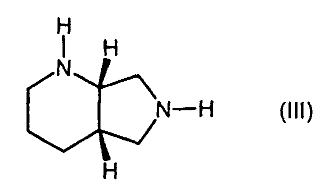 if appropriate in the presence of a base
in ethanol in a mixture with a polar aprotic diluent;
or
by heating CCDC of an unknown modification in a diluent, such as ethanol, propanol or isopropanol or in a mixture of these alcohols, with a polar aprotic diluent, if appropriate in the presence of a base, subsequently cooling the mixture and isolating CCDC of the crystal modification B.

4. Process for preparing CCDC of the modification B according to any of Claims 1 to 3, **characterized in that** 7-halogeno-8-cyano-1-cyclopropyl-6-fluoro-1,4-dihydro-4-oxo-3-quinolinecarboxylic acid of the formula (II) 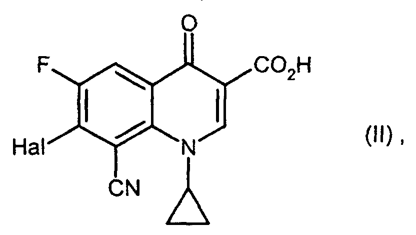 in which
Hal represents fluorine or, preferably, represents chlorine
and (1S,6S)-2,8-diazabicyclo[4.3.0]nonane of the formula (III) 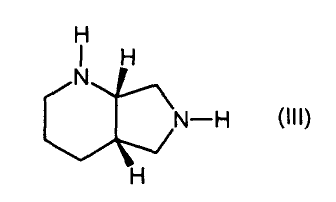 are reacted, if appropriate in the presence of a base, in
ethanol in a mixture with a polar aprotic diluent,
or
that CCDC of an unknown modification is heated in a diluent, such as ethanol, propanol or isopropanol or in a mixture of these alcohols, with a polar aprotic diluent, if appropriate in the presence of a base, the mixture is subsequently cooled and CCDC of the crystal modification B is isolated.

5. Process for preparing CCDC of the modification B according to Claim 4, **characterized in that** the polar aprotic solvent used is N-methylpyrrolidone, dimethylformamide or sulpholane.

6. Process for preparing CCDC of the modification B according to Claim 4 or 5, **characterized in that**, if the solvent used is ethanol, N-methylpyrrolidone, dimethylformamide or sulpholane are employed as further solvents.

7. Process for preparing CCDC of the modification B according to any of Claims 4 to 6, **characterized in that**, if the solvent used is ethanol, N-methylpyrrolidone is employed as further solvent.

8. Process for preparing CCDC of the modification B according to any of Claims 4 to 7, **characterized in that** the base used is trimethylamine, triethylamine or ethyl-diisopropylamine.

9. Medicament, **characterized in that** it comprises, in addition to customary auxiliaries and excipients, CCDC of the modification B according to any of Claims 1 to 3.

10. The use of CCDC of the modification B according to any of Claims 1 to 3 for preparing medicaments.

11. The use of CCDC of the modification B according to Claim 10 for preparing antibacterial medicaments.

## Revendications

1. Acide 8-cyano-1-cyclopropyl-7-(1S,6S-2,8-diazabicyclo[4.3.0]nonane-8-yl)-6-fluoro-1,4-dihydro-4-oxo-3-quinoléinecarboxylique (CCDC) de la forme cristalline B, **caractérisé en ce qu'**il présente un diffractogramme X de poudre avec les positions de réflexion (2 thêta) de haute et moyenne intensité suivantes :

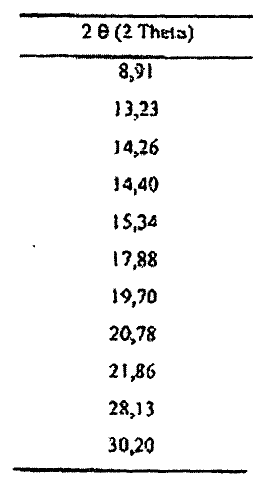

2. Acide 8-cyano-1-cyclopropyl-7-(1S,6S-2,8-diazabicyclo[4.3.0]nonane-8-yl)-6-fluoro-1,4-dihydro-4-oxo-3-quinoléinecarboxylique (CCDC) de la forme cristalline B, **caractérisé en ce qu'**il présente un diffractogramme X de poudre avec les positions de réflexion (2 thêta) de haute et moyenne intensité suivantes : 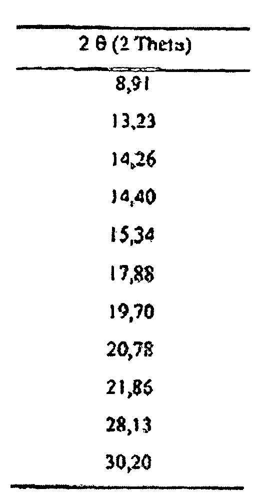 et possède un point de fusion déterminé par thermo-analyse différentielle de 243° à 245°C.

3. Acide 8-cyano-1-cyclopropyl-7-(1S,6S-2,8-diazabicyclo[4.3.0]nonane-8-yl)-6-fluoro-l,4-dihydro-4-oxo-3-quinoléinecarboxylique (CCDC) de la forme cristalline B, obtenu par réaction d'un acide 7-halogéno-8-cyano-1-cyclopropyl-6-fluoro-l,4-dihydro-4-oxo-3-quinolonecarboxylique de formule (II) 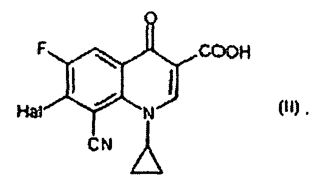 dans laquelle
Hal représente le fluor ou plus avantageusement le chlore, et du (1S,6S)-2,8-diazabicyclo[4.3.0]nonane de formule (III) 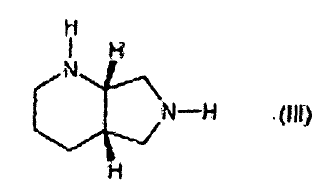 éventuellement en présence d'une base
dans l'éthanol en mélange avec un diluant aprotique polaire ;
ou bien
par chauffage de CCDC de forme inconnue dans un diluant tel que l'éthanol, le propanol ou l'isopropanol ou dans un mélange de ces alcools avec un diluant aprotique polaire, éventuellement en présence d'une base, puis refroidissement du mélange et isolement du CCDC de la forme cristalline B.

4. Procédé de production de CCDC de la forme B suivant l'une des revendications 1 à 3, **caractérisé en ce qu'**on fait réagir un acide 7-halogéno-8-cyano-1-cyclopropyl-6-fluoro-1,4-dihydro-4-oxo-3-quinolonecarboxylique de formule (II) 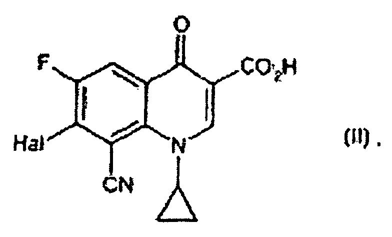 dans laquelle
Hal représente le fluoro ou plus avantageusement le chlore, et le (1S,6S)-2,8-diazabicylo[4.3.0]nonane de formule (III) 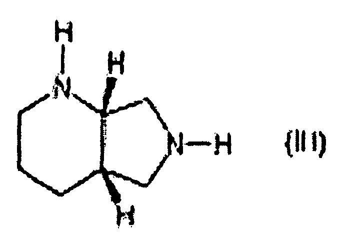 éventuellement en présente d'une base
dans l'éthanol en mélange avec un diluant aprotique polaire ou bien
on chauffe un CCDC de forme inconnue dans un diluant tel que l'éthanol, le propanol ou l'isopropanol ou dans un mélange de ces alcools avec un diluant aprotique polaire, éventuellement en présence d'une base, puis on refroidit le mélange et on isole le CCDC de la forme cristalline B.

5. Procédé de production de CCDC de la forme B suivant la revendication 4, **caractérisé en ce qu'**on utilise comme solvant aprotique polaire la N-méthylpyrrolidone, le diméthylformamide ou le sulfolane.

6. Procédé de production de CCDC de la forme B suivant la revendication 4 ou 5, **caractérisé en ce que** lorsque l'on utilise l'éthanol comme solvant, on utilise comme autres solvants la N-méthylpyrrolidone, le diméthylformamide ou le sulfolane.

7. Procédé de production de CCDC de la forme B suivant l'une des revendications 4 à 6, **caractérisé en ce que** lorsque l'on utilise l'éthanol comme solvant, on utilise la N-méthylpyrrolidone comme autre solvant.

8. Procédé de production de CCDC de la forme B suivant l'une des revendications 4 à 7, **caractérisé en ce qu'**on utilise comme base la triméthylamine, la triéthylamine ou l'éthyldiisopropylamine.

9. Médicament, **caractérisé en ce qu'**il contient, à côté de substances auxiliaires et de supports habituels, du CCDC de la forme B suivant l'une des revendications 1 à 3.

10. Utilisation de CCDC de la forme B suivant l'une des revendications 1 à 3 pour la préparation de médicaments.

11. Utilisation de CCDC de la forme B suivant la revendication 10 pour la préparation de médicaments antibactériens.
